Europäisches Patentamt

European Patent Office

Office européen des brevets

(19)

(11) Publication number: **0 179 857 B1**

(12) **EUROPEAN PATENT SPECIFICATION**
**published in accordance with Art.**
**158(3) EPC**

(45) Date of publication of patent specification: **27.02.91**  (51) Int. Cl.⁵: **C07C 2/00**

(21) Application number: **85902266.7**

(22) Date of filing: **11.04.85**

(86) International application number:
**PCT/US85/00649**

(87) International publication number:
**WO 85/04865 (07.11.85 85/24)**

(54) **METHANE CONVERSION.**

(30) Priority: **16.04.84 US 600659**
                **16.04.84 US 600668**

(43) Date of publication of application:
**07.05.86 Bulletin  86/19**

(45) Publication of the grant of the patent:
**27.02.91 Bulletin  91/09**

(84) Designated Contracting States:
**BE DE FR NL**

(56) References cited:
**GB-A- 258 608**      **US-A- 4 199 533**
**US-A- 4 205 194**   **US-A- 4 239 658**
**US-A- 4 443 645**   **US-A- 4 443 648**
**US-A- 4 444 984**

**N; JOURNAL OF CATALYSIS, ISSUED 1982,**
**G.E. KELLER AND M.M. BHASIN, SYNTHESIS**
**OF ETYLENE VIA OXIDATIVE COUPLING OF**
**METHANE, SEE PAGES 9-18.**

(73) Proprietor: **ATLANTIC RICHFIELD COMPANY**
**515 South Flower Street**
**Los Angeles California 90071(US)**

(72) Inventor: **JONES, C. Andrew**
**24 Clearbrook Road**
**Newtown Square, PA 19073(US)**
Inventor: **SOFRANKO, John, A.**
**Box 197, Line Road, R.D. 4**
**Malvern, PA 19355(US)**
Inventor: **WITHERS, Howard, P.**
**Valley View Apartments, Yorktown 23**
**Pottstown, PA 19464(US)**

(74) Representative: **Cropp, John Anthony David et**
**al**
**MATHYS & SQUIRE 10 Fleet Street**
**London, EC4Y 1AY(GB)**

**EP 0 179 857 B1**

N, JOURNAL OF THE CHINESE CHEMICAL
SOCIETY, TRELANT FANG AND CHUIN- TIH
YEH, CATALYTIC PYRLYSIS OF METHANE,
SEE PAGES 265-273

## Description

BACKGROUND OF THE INVENTION

This invention relates to the synthesis of hydrocarbons from a methane source. A particular application of this invention is a method for converting natural gas to more readily transportable material.

A major source of methane is natural gas. Other sources of methane have been considered for fuel supply, e.g., the methane present in coal deposits or formed during mining operations. Relatively small amounts of methane are also produced in various petroleum processes.

The composition of natural gas at the wellhead varies but the major hydrocarbon present is methane. For example, the methane content of natural gas may vary within the range from about 40 to about 95 volume percent. Other constituents of natural gas include ethane, propane, butanes, pentane (and heavier hydrocarbons), hydrogen sulfide, carbon dioxide, helium and nitrogen.

Natural gas is classified as dry or wet depending upon the amount of condensable hydrocarbons contained in it. Condensable hydrocarbons generally comprise $C_3+$ hydrocarbons although some ethane may be included. Gas conditioning is required to alter the composition of wellhead gas, processing facilities usually being located in or near the production fields. Conventional processing of wellhead natural gas yields processed natural gas containing at least a major amount of methane.

Large scale use of natural gas often requires a sophisticated and extensive pipeline system. Liquefaction has also been employed as a transportation means, but processes for liquefying, transporting, and revaporizing natural gas are complex, energy-intensive and require extensive safety precautions. Transport of natural gas has been a con tinuing problem in the exploitation of natural gas resources. It would be extremely valuable to be able to convert methane (e.g., natural gas) to more readily handleable or transportable products. Moreover, direct conversion to olefins such as ethylene or propylene would be extremely valuable to the chemical industry.

Recently, it has been discovered that methane may be converted to higher hydrocarbons (e.g., ethane, ethylene and higher homologs) with minimal formation of carbon oxides by contacting methane with a reducible metal oxide as a selective oxygen source. As the methane is converted to hydrocarbon products and co-product water, the active oxygen of the metal oxide is depleted, resulting in a reduced metal oxide. The reduced metal oxide is relatively inactive for the oxidative conversion of methane but active oxygen may be replaced by regeneration of a reducible metal oxide. Such regeneration is accomplished by reoxidation of the reduced metal oxide.

Reducible oxides of several metals have been identified which are capable of converting methane to higher hydrocarbons. Oxides of manganese, tin, indium, germanium, lead, antimony and bismuth are particularly useful.

Greater amounts of $C_3+$ hydrocarbon products may be obtained by effecting the contact under elevated pressure [e.g. 2-100 bar (atmospheres)].

U.S. Patent 4,499,322 discloses and claims a process for the conversion of methane to higher hydrocarbons which comprises contacting methane with an oxidative synthesising agent containing a promoting amount of alkali metal and/or compounds thereof.

U.S. Patent 4,495,374 discloses and claims a process for the conversion of methane to higher hydrocarbons which comprises contacting methane with an oxidative synthesising agent containing a promoting amount of alkaline earth metal and/or compounds thereof.

Reducible oxides of cerium, praseodymium, and terbium have also been found to be effective for the conversion of methane to higher hydrocarbons, especially when the rare earth component is associated with an alkali or alkaline earth metal component. See concurrently filed European patent application 85902267.5 (published as EP-A-0179131).

The same application discloses and claims a process for the conversion of methane to higher hydrocarbons which comprises contacting methane with a contact solid comprising a reducible oxide of iron or ruthenium and at least one member of the group consisting of alkali metals, alkaline earth metals, and compounds thereof.

As noted, the reaction products of such processes are mainly ethylene, ethane and other light hydrocarbons, carbon oxides, coke and water. It would be beneficial in these processes to reduce selectivities to carbon oxides and coke and to increase methane conversions to the desired hydrocarbon products.

GB-A-258608 describes a process for obtaining aromatic hydrocarbons from methane-containing gases by passing the gas over a catalyst of specified kind. Amongst those materials listed as being suitable as catalysts are compounds of Se, Te or TI, or active silica or active-charcoal or mixtures of these substances.

The concept of using reducible oxides of metals, whether alone or with promoting substances, is, however, not recognised.

The Journal of Catalysis, 1982, pages 9 to 18 describes the formation of ethylene and ethane by the catalytic oxidative coupling of methane. A number of metal oxides are examined for catalytic activity.

US-A-4199533 describes another method for the conversion of methane to higher molecular weight hydrocarbons, in which a mixture of chlorine and methane-containing gas is reacted in specific ratios and under specific temperature conditions. HCl is formed in significant quantities as a by-product.

## SUMMARY OF THE INVENTION

The present invention relates to an improved method of converting methane to higher hydrocarbons in which the contacting of the methane with a contact solid comprising reducible metal oxide is effected in the presence of a promoting amount of at least one halogen and/or chalcogen component.

According to the present invention there is provided a method for converting methane to higher hydrocarbon products which comprises contacting a gas comprising methane at a temperature in the range of $500°C$ to $1000°C$ with a contact solid comprising at least one reducible oxide (as herein defined) of at least one metal which oxide when contacted with methane at a temperature in said range is reduced and produces higher hydrocarbon products and water, wherein the contacting is effected in the presence of at least one promoter selected from halogens, chalcogens and compounds thereof, said chalcogens being selected from sulfur, selenium and tellurium.

In accordance with one embodiment, the method comprises the steps of:

(a) contacting a gas comprising methane with a contact solid comprising at least one said reducible oxide of at least one metal under conditions at which said at least one reducible oxide is reduced to form solids comprising reduced metal oxide and produces higher hydrocarbon products and water;

(b) recovering higher hydrocarbons;

(c) at least periodically contacting solids comprising reduced metal oxide with an oxygen containing gas to regenerate solids comprising reducible metal oxide;

(d) contacting a gas comprising methane with regenerated solids produced in step (c) in the manner specified in step (a); and

(e) at least periodically contacting said solids comprising reduced metal oxide and/or said regenerated solids comprising reducible metal oxide with at least one promoter source selected from halogen sources and chalcogen sources.

In accordance with another embodiment, the method includes the further steps of:

(a) recovering higher hydrocarbons produced by said contacting;

(b) at least periodically contacting solids comprising reduced metal oxide, and obtained by said reduction of said at least one reducible oxide, with an oxygen-containing gas to regenerate solids comprising reducible metal oxide;

(c) contacting a gas comprising methane with regenerated solids produced in step (b) in the manner specified in step (a); and

(d) at least perodically contacting said solids comprising reduced metal oxide and/or said regenerated solids comprising reducible metal oxide with at least one promoter source selected from halogen sources and chalcogen sources to incorporate additional promoter into said solids.

The contact solid may further comprise at least one member selected from alkali metals, alkaline earth metals and compounds thereof.

Halogens are selected from fluorine, chlorine, bromine and iodine. Preferred halogen promoters are chlorine, bromine, and compounds thereof. Chlorine and compounds of chlorine are particularly preferred. Chalcogens are selected from sulfur, selenium and tellurium. Preferred chalcogen promoters are sulfur and compounds thereof. Reducible oxides of manganese are particularly preferred.

In addition to methane the feedstock employed in the method of this invention may contain other hydrocarbon or non-hydrocarbon components. The methane content of the feedstock, however, will typically be within the range of 40 to 100 vol. %, preferably within the range of 80 to 100 vol. %, more preferably within the range of 90 to 100 vol. %.

The solid which is contacted with methane in the method of the present invention comprises at least one oxide of at least one metal, which oxide when contacted with methane at temperatures selected within the range of 500 to $1000°C$ produces higher hydrocarbon products, co-product water and reduced metal oxide. The contact solid thus contains at least one reducible oxide of at least one metal. The term "reducible" identifies those oxides of metals which are reduced by the methane contact. The term "oxide(s) of metal(s)", as used herein, means (1) one or more metal oxides (i.e., compounds described by the general formula $M_x O_y$ wherein M is a metal and the subscripts x and y designate the relative atomic proportions of metal and oxygen in the composition) and/or (2) one or more oxygen-containing metal compounds, provided that such oxides and compounds have the capability of performing to produce higher hydrocarbon products as set forth herein.

Effective agents for the conversion of methane to higher hydrocarbons have previously been found to comprise reducible oxides of metals selected from the group consisting of manganese, tin, indium, germanium, antimony, lead, bismuth and mixtures thereof.

Reducible oxides of cerium, praseodymium, and terbium have also been found to be effective for the conversion of methane to higher hydrocarbons, particularly when the rare earth component is associated with an alkali or alkaline earth metal component.

Reducible oxides of iron and ruthenium are also effective for the conversion of methane to higher hydrocarbons, particularly when associated with an alkali or alkaline earth metal.

In one embodiment of the invention, the contact solid employed in the process of the present invention contains, in addition to the reducible metal oxide component, at least one promoter selected from halogens, chalcogens and compounds thereof. The atomic ratio in which these materials are combined to form the contact solid is not narrowly critical. However, the preferred atomic ratio of the reducible oxide component (expressed as the metal, e.g., Mn) to the halogen or chalcogen component (expressed as the halogen or chalcogen, e.g., S) may range up to about 1:5, more preferably the ratio is within the range of about 1:3 to 1000:1.

Methane conversion may be improved by introducing a halogen or chalcogen source at least periodically into the process. Methane conversion may also be improved by initially using a halogen or chalcogen-containing compound to prepare the contact solid. Regardless of how the halogen/chalcogen component is introduced into the process, the solid composition will contact such halogen/chalcogen, and will retain the halogen/chalcogen for a period of time after introduction of the halogen/chalcogen is terminated. The retention of the halogen/chalcogen and/or the retention of the beneficial effects caused by the presence of the halogen/chalcogen is a particularly advantageous feature of the process of this invention.

According to a distinct, highly preferred aspect of this invention, it has been found that the presence of at least one alkali metal component prolongs the period of such retention of the beneficial effects caused by halogen/chalcogen addition. Sodium and/or compounds thereof are a particularly preferred alkali metal component of this distinct aspect of the invention. Other suitable alkali metal components are lithium and potassium and compounds thereof.

The contact solid may optionally contain at least one phosphorus component. The amount of phosphorus contained in the contact solid is again not narrowly critical. The atomic ratio of phosphorus to the reducible oxide component (expressed as the metal, e.g., Mn) is preferably less than about 2:1. More preferably, this ratio is within the range of about 0.1-0.5:1.

One preferred contact solid used in the process of thin invention may be further expressed by the following empirical formula:

$$A_a X_b P_c O_d$$

wherein A is selected from Mn, Sn, In, Ge, Pb, Sb, Bi and mixtures thereof; X is selected from F, Cl, Br, I, S, Se, Te and mixtures thereof; a to d indicate the atomic ratio of each component; and when a is 10, b is within the range of about 0.01-30, c is within the range of about 0-20, and d has a value which is determined by the valence and proportions of the other elements present.

The foregoing components of the contact solid may be associated with other support materials such as silica, alumina, titania, magnesia, zirconia and combinations thereof. When employing agents containing rare earth components--oxides of Ce, Pr, and Tb--the rare earth oxides preferably serve as supports.

Reducible oxides of manganese have been found to be particularly desirable for methane conversion according to the method of the present invention. Particularly preferred agents comprise silica- and magnesia-supported halogen-promoted solids containing oxides of manganese and sodium.

The contact solid can be prepared by any suitable method. Conventional methods such as precipitation, coprecipitation, impregnation or dry mixing can be used. supported solids may be prepared by methods such as adsorption, impregnation, precipitation, coprecipitation, and dry mixing. When phosphorus is incorporated into the agent, it is desirable to provide it in the form of a phosphate of an alkali metal.

A suitable method of preparation is to impregnate a support with solutions of the desired metals. Suitable compounds useful for impregnation include the acetates, acetylacetonates, oxides, carbides, carbonates, hydroxides, formates, oxalates, nitrates, phosphates, sulfates, sulfides, tartrates, fluorides, chlorides, bromides, or iodides. After impregnation the preparation is dried to remove solvent and the dried solid is calcined, preferably in air, at a temperature within the range of about 300 to 1200°C. Particular calcination temperatures will vary depending upon the particular metal compound or compounds employed.

Halogen components may conveniently be incorporated into the contact solid either before or, after calcination of the metal-containing composite, provided that if incorporated before calcination halogen remains in the contact solid after calcination.

A suitable method of incorporation is to impregnate the composite with solutions containing the desired halogens. Suitable compounds for impregnation include $NH_4Cl$ , $NaCl$ , $HCl$ and $MCl_x$ . Another suitable method of incorporation is to contact the composite with a halogen source.

The halogen source may be any of a wide number of materials. The source may be either free halogen gas or a compound of halogen. Suitable sources of halogen include hydrogen iodide, hydrogen bromide, and hydrogen chloride; ammonium halides, aliphatic halides such as methyl chloride, methylene chloride, ethyl chloride, amyl chloride and allyl chloride; cycloaliphatic halides such as cyclohexyl halide; halogen substituted aliphatic acids such as methyl amine hydrochloride.

Mixtures of various halogen sources may be used. The presently preferred halogen sources are free halogen gas, aliphatic halides and hydrogen halides.

Chalcogen components may conveniently be incorporated into the contact solid either before or after calcination of the metal-containing composite, provided that if incorporated before calcination chalcogen remains in the contact solid after calcination. A suitable method of incorporation is to impregnate the composite with solutions containing the desired chalcogens. Another suitable method of incorporation is to contact the composite with a chalcogen source.

The chalcogen source may be any of a wide number of materials. The source may be either free chalcogen gas or a chalcogen compound. Suitable sources of chalcogen include hydrogen sulfide, hydrogen selenide, and hydrogen telluride; chalcogen oxides such as $SO_2$; ammonium chalcogenides; aliphatic chalcogenides such as methyl sulfide, methylene sulfide, ethyl sulfide, amyl sulfide and allyl sulfide; cycloaliphatic chalcogenides such as cyclohexyl sulfide; chalcogen substituted aliphatic acids ; and organic amine chalcogenide salts. Mixtures of various chalcogen sources may be used. The presently preferred chalcogen sources are aliphatic chalcogenides, hydrogen chalcogenides, and chalcogen oxides.

Regardless of how the components of the contact solid are combined, the composite will generally be dried and calcined at elevated temperatures prior to use in the process of this invention.

Preferably, methane and oxygen are contacted with the solid in the substantial absence of catalytic-ally effective nickel, noble metals and compounds thereof (i.e., nickel, rhodium, palladium, silver, osmium, iridium, platinum and gold) to minimize the deleterious catalytic effects thereof. These metals, when contacted with methane at the temperatures employed in the method of the present invention, tend to promote coke formation, and the metal oxides tend to promote the formation of combustion products rather than the desired hydrocarbons. The term "catalytically effective" is used herein to identify that quantity of one or more of nickel and of the noble metals and compounds thereof which substantially changes the distribution of products obtained in the method of this invention relative to such contacting in the absence of such metals and compounds thereof.

Operating temperatures for the method of this invention are within the range of 500 to 1000$^\circ$ C. If reducible oxides of metals such as In, Ge, or Bi are present in the solid, the particular temperature selected may depend, in part, on the particular reducible metal oxide(s) employed. Thus, reducible oxides of certain metals may require operating temperatures below the upper part of the recited range to minimize sublimation or volatilization of the metals (or compounds thereof) during methane contact. Examples are: (1) reducible oxides of indium, (operating temperatures will preferably not exceed about 850$^\circ$ C); (2) reducible oxides of germanium (operating temperatures will preferably not exceed about 850$^\circ$ $-$ C); and (3) reducible oxides of bismuth (operating temperatures will preferably not exceed about 850$^\circ$ C).

Operating pressures for the methane contacting step are not critical to the presently claimed invention. However, both general system pressure and partial pressure of methane have been found to affect overall results. Preferred operating pressures are within the range of about 1 to 100 bar (atmospheres), more preferably within the range of about 1 to 30 bar (atmospheres).

Contacting methane and a reducible metal oxide to form higher hydrocarbons from methane also produces a reduced metal oxide and co-product water. The exact nature of the reduced metal oxides are unknown, and so are referred to herein as "reduced metal oxides". Regeneration of a reducible metal oxide is readily accomplished by contacting such reduced materials with oxygen (e.g., an oxygen-containing gas such as air) at elevated temperatures, preferably at a temperature selected within the range of 300 to 1200$^\circ$ C, the particular temperature selected depending on the metal(s) included in the solid.

When contacting methane with a promoted contact solid according to the present invention, a single reactor apparatus containing a fixed bed of solids may be used with intermittent or pulsed flow of a first gas comprising methane and a second gas comprising oxygen (e.g., oxygen, oxygen diluted with an inert gas,

EP 0 179 857 B1

or air, preferably air). The methane contacting step and the oxygen contacting step may also be performed in physically separate zones with solids recirculating between the two zones.

Thus, a suitable method for synthesizing hydrocarbons from a methane source comprises: (a) contacting a gas comprising methane and a promoted contact solid comprising at least one reducible oxide of at least one metal and a halogen or chalcogen promoter to form higher hydrocarbon products, co-product water, and reduced metal oxide; (b) removing solids comprising reduced metal oxide from the first zone and contacting the reduced solids in a second zone with an oxygen-containing gas to form solids comprising a reducible metal oxide; and (c) returning the contact solid produced in the second zone to the first zone. The steps are preferably repeated at least periodically, and more preferably the steps are continuous. In one more preferred embodiment solids are continuously circulated between at least one methane-contact zone and at least one oxygen-contact zone.

Promoted contact solids comprising a reducible metal oxide which are contacted with methane may be maintained as fluidized, ebullating, or entrained beds of solids. Preferably methane is contacted with a fluidized bed of solids.

Similarly, solids comprising reduced metal oxide which are contacted with oxygen may be maintained as fluidized, ebullating or entrained beds of solids. Preferably oxygen is contacted with a fluidized bed of solids.

In one more preferred embodiment of the present invention, methane feedstock and promoted contact solids are continuously introduced into a methane contact zone maintained at synthesizing conditions. Synthesizing conditions include the temperatures and pressures described above. Gaseous reaction products from the methane contact zone (separated from entrained solid) may be further processed--e.g., they may be passed through a fractionating system wherein the desired hydrocarbon products are separated from uncoverted and combustion products. Unconverted methane may be recovered and recycled to the methane contact zone.

Solids comprising reduced metal oxide are contacted with oxygen in an oxygen contact zone for a time sufficient to oxidize at least a portion of the reduced oxide to produce a reducible metal oxide and to remove, i.e., combust, at least a portion of any carbonaceous deposit which may form on the solids in the methane contact zone. The conditions of the oxygen contact zone will preferably include a temperature selected within the range of 300 to 1200°C, pressures of up to about 30 atmospheres, and average particle contact time within the range of 1 to 120 minutes. Sufficient oxygen is preferably provided to oxidize all reduced metal oxide to produce a reducible oxide and to completely combust any carbonaceous deposit material desposited on the solids. At least a portion of the promoted contact solids which are produced in the oxygen contact zone are returned to the methane contact zone.

The rate of solids withdrawal from the methane contact zone is desirably balanced with the rate of solids passing from the oxygen contact zone to the methane contact zone so as to maintain a substantially constant inventory of particles in the methane contact zone, thereby enabling steady state operation of the synthesizing system.

When halogen- or chalcogen-promoted contact solids are employed in the methane conversion process of this invention, it has been found that the enhanced methane conversion activity and selectivity to higher hydrocarbons attributable to the halogen/chalcogen component is dissipated over time. Therefore, additional halogen/chalcogen component must be incorporated into the contact solid as the cycle is repeated in order to maintain the desirable results obtained by this invention.

It is within the scope of this invention to incorporate additional halogen or chalcogen into the contact solid by any of the methods described in the foregoing discussion concerning preparation of the promoted contact solid. Preferably, the solid is periodically contacted with a halogen or chalcogen source. Such contact preferably occurs regularly and repeatedly during the cycle comprising methane contact and oxygen regeneration.

For example, when employing a process wherein: (1) a gas comprising methane and solids comprising at least one reducible oxide of at least one metal are continuously introduced and contacted in a first zone (preferably containing a fluidized bed of solids) to produce higher hydrocarbons and (2) an oxygen-containing gas and reduced metal oxides are contacted in a second zone (also preferably containing a fluidized bed of solids) to regenerate reducible metal oxides); a halogen/chalcogen source may be periodically added either to the gas comprising methane being fed to the first zone or to the oxygen-containing gas being fed to the second zone. It is also possible to periodically add the halogen/chalcogen source to at least a portion of the solids as they recirculate between the two zones.

When employing a fixed bed reactor system, a halogen/chalcogen source may be periodically added with: (1) the gas comprising methane, preheated to reaction temperature, as it is introduced to reactors during the methane conversion portion of the fixed bed

7

process cycle; (2) the gas comprising methane as it is being introduced to reactors during the methane preheat portion of the process cycle; (3) the oxygen-containing gas as it is introduced to reactors during the regeneration portion of the process cycle; (4) the purge gas introduced to the reactors between the methane preheat and regeneration portions of the process cycle; and (5) the purge gas introduced to the reactors between the regeneration and methane conversion portions of the process cycle. As will be apparent to one skilled in the art, the process and apparatus may also be modified to provide that each reactor is periodically isolated from other process streams and contacted with a halogen/chalcogen source.

This invention is further illustrated by reference to the following examples. Experimental results reported below include conversions and selectivities calculated on a carbon mole basis.

EXAMPLE 1

A chlorine-promoted contact solid comprising a reducible oxide of tin was prepared by impregnating tin tartrate, provided as an aqueous solution containing 7 wt. % hydrochloric acid, on Houdry (Registered Trade Mark) HSC 534 silica, the amount of tin provided being sufficient to yield a solid containing 5 wt. % Sn/ $SiO_2$. The solids were dried at 110° C for 4 hours and then calcined in air at 700° C for 16 hours. A quartz tube reactor (12mm. inside diameter) was packed with 10 ml. of the calcined solids. The reactor was brought up to reaction temperature (700° C) under a flow of nitrogen. A feed of 100% methane was then contacted with the solids at about atmospheric pressure and a GHSV (gas hourly space velocity) of 600 hrs.$^{-1}$. Instantaneous samples of the effluent were taken throughout the methane contact run and analyzed by gas chromatography and gas chromatography mass spectroscopy. Results are reported below in Table I.

## TABLE 1

| Run time (min) | % Conversion | % Selectivity | | | | | |
|---|---|---|---|---|---|---|---|
| | | $C_2H_4$ | $C_2H_6$ | $C_3$ | $C_{4-7}$ | CO | $CO_2$ |
| Instantaneous Results | | | | | | | |
| 0.5 | 1.42 | 22.2 | 33.8 | 8.7 | 16.3 | 18.8 | -- |
| 1.0 | 0.27 | 31.4 | 31.4 | 10.9 | 25.8 | 0.38 | -- |
| 2.0 | 0.39 | 33.2 | 31.6 | 10.9 | 24.1 | --- | -- |
| Cumulative Results | | | | | | | |
| 15 | 0.22 | 32.5 | 20.1 | 9.7 | 16.2 | 13.4 | 7.8 |

At the end of the methane-contact run described above, the reactor was flushed with nitrogen and the solids were regenerated under a flow of air at 700° C. The reactor was then again flushed with nitrogen and the feed of 100% methane was reintroduced to the reactor under the same conditions employed in the first run. Results are reported below in Table II.

8

## TABLE II

| Run time (min) | % Conversion | % Selectivity | | | | | |
|---|---|---|---|---|---|---|---|
| | | $C_2H_4$ | $C_2H_6$ | $C_3$ | $C_{4-7}$ | CO | $CO_2$ |
| Instantaneous Results | | | | | | | |
| 0.5 | 3.18 | 7.2 | 13.7 | 0.2 | 0.8 | 15.5 | 62.3 |
| 1.0 | 1.27 | 8.8 | 17.2 | 0.5 | 2.7 | 24.7 | 45.7 |
| 2.0 | 0.26 | 34.3 | 52.7 | 3.6 | 9.2 | --- | -- |
| Cumulative Results | | | | | | | |
| 15 | 0.23 | 29.9 | 29.9 | 3.4 | 8.6 | 11.0 | 16.9 |

Several more cycles of methane-contact/re__ generation were performed using the contact solid described above.

During the 5th methane contact run (700° C, 100% methane feed, 600 GHSV), the results shown below in Table III were obtained.

## TABLE III

| Run time (min) | % Conversion | % Selectivity | | | | | |
|---|---|---|---|---|---|---|---|
| | | $C_2H_4$ | $C_2H_6$ | $C_3$ | $C_{4-7}$ | CO | $CO_2$ |
| Instantaneous Results | | | | | | | |
| 0.5 | 3.51 | 0.21 | 1.03 | 0 | 0.19 | 2.05 | 78.1 |
| 1 | 0.92 | 0.67 | 3.13 | 0 | 0.51 | 0 | 95.7 |
| 2 | 0.22 | 4.7 | 14.1 | 0.48 | 0.38 | 0 | 80.3 |
| Cumulative Results | | | | | | | |
| 15 | 0.19 | 4.99 | 12.5 | 1.02 | 6.18 | 41.8 | 33.5 |

The spent solid from the 5th run was re__ oxidized as described above. The reoxidized solid was then wetted with an aqueous solution containing 16% HCl, and the wetted solid was dried at 110° C for 4 hours. These solids were again placed into a quartz tube reactor and, following the procedures described above, was contacted with methane at 800° C at a GHSV of 600 hrs.$^{-1}$. Results are shown below in Table IV.

## TABLE IV

| Run time (min) | % Conversion | % Selectivity | | | | | |
|---|---|---|---|---|---|---|---|
| | | $C_2H_4$ | $C_2H_6$ | $C_3$ | $C_{4-7}$ | CO | $CO_2$ |
| Instantaneous Results | | | | | | | |
| 0.5 | 4.31 | 31.1 | 13.8 | 4.6 | 4.1 | 21.5 | 24.9 |
| 1.0 | 1.94 | 39.4 | 35.2 | 3.2 | 2.3 | 11.2 | 8.7 |
| 2.0 | 0.93 | 34.8 | 38.6 | 2.6 | 2.0 | 22.1 | 0.0 |
| Cumulative Results | | | | | | | |
| 15 | 0.88 | 35.8 | 29.9 | 2.6 | 1.3 | 30.4 | 0.0 |

9

At the end of the methane contact run described in Table IV, the solids were regenerated and then contacted again with methane at 800° C and a GHSV of 600 hr.$^{-1}$. Results are shown below at Table V.

## TABLE V

| Run time (min) | % Conversion | % Selectivity | | | | | |
|---|---|---|---|---|---|---|---|
| | | $C_2H_4$ | $C_2H_6$ | $C_3$ | $C_{4-7}$ | CO | $CO_2$ |
| Instantaneous Results* | | | | | | | |
| 0.5 | 8.04 | 15.1 | 15.0 | 1.0 | 0.7 | 9.0 | 59.2 |
| 1.0 | 17.60 | 22.0 | 55.7 | 3.8 | 3.1 | 1.3 | 14.2 |
| Cumulative Results | | | | | | | |
| 15 | 0.97 | 25.2 | 25.0 | 1.7 | 1.4 | 29.0 | 17.8 |

*Instantaneous results at a run time of 2.0 minutes are omitted because of analytical problems.

## EXAMPLE 2

A chlorine-promoted contact solid comprising a reducible oxide of manganese was prepared by impregnating manganese, provided as an aqueous solution of manganese acetate, on Houdry HSC 534 silica, the amount of manganese provided being sufficient to yield a solid containing 15 wt. % $Mn/SiO_2$. The impregnated solids were dried at 100° C for four hours and then calcined in air at 700° C for 16 hours. The calcined solids (4.34 gms). were placed in 7 ml. $H_2O$ and 12 drops of concentrated HCl was added to the mixture. The solid was then dried at 100° C. A quartz tube reactor (12 mm. inside diameter) was charged with 10 ml. of the HCl-impregnated solid. The reactor was brought up to reactor temperature (750° C) under a flow of nitrogen. A feed of 100% methane was then contacted with the solids at about atmospheric pressure and a GHSV of 600 hrs.$^{-1}$. Results are reported below in Table VI.

## TABLE VI

| Run time (min) | % Conversion | % Selectivity | | | | | |
|---|---|---|---|---|---|---|---|
| | | $C_2H_4$ | $C_2H_6$ | $C_{3-7}$ | CO | $CO_2$ | RX* |
| Instantaneous Results | | | | | | | |
| 0.5 | 51.9 | 46.7 | 2.2 | 21.8 | 0.2 | 22.2 | 6.8 |
| 1.0 | 7.2 | 18.6 | 33.1 | 35.9 | 0.8 | 2.3 | 9.1 |
| 2.0 | 0.26 | 26.9 | 73.1 | 0 | 0 | 0 | trace |
| 4.0 | 0.23 | 30.4 | 69.6 | 0 | 0 | 0 | --- |
| Cumulative Results | | | | | | | |
| 15 | 1.8 | 30.1 | 8.37 | 16.2 | 11.7 | 29.0 | 4.57 |

*Halogenated hydrocarbons such as methyl chloride, methylene chloride, ethylene chloride and chlorobenzene.

At the end of the methane contact run described above, the reactor was flushed with nitrogen and the solids were regenerated under a flow of air at 750°C. The reactor was then again flushed with nitrogen and a feed of 100% methane was reintroduced into the reactor under the same conditions employed in the first run. Results are reported below in Table VII. No halogenated products were detected in the reactor effluent.

## TABLE VII

| Run time (min) | % Conversion | % Selectivity | | | | |
|---|---|---|---|---|---|---|
| | | $C_2H_4$ | $C_2H_6$ | $C_{3-7}$ | CO | $CO_2$ |
| Instantaneous Results* | | | | | | |
| 0.5 | 7.65 | 22.0 | 57.3 | 8.6 | 0.5 | 11.5 |
| 2.0 | 3.38 | 13.5 | 79.6 | 4.0 | 0.0 | 2.9 |
| 4.0 | 1.82 | 9.9 | 87.0 | 2.6 | 0.0 | 0.4 |
| Cumulative Results | | | | | | |
| 15 | 2.00 | 11.6 | 75.1 | 7.6 | 0.0 | 5.8 |

*Instantaneous results at a run time of 1.0 minutes are omitted because of analytical problems.

## COMPARATIVE EXAMPLE A

A contact solid consisting of 15 wt. % Mn/ SiO₂ was prepared as described in Example 2 except that the HCl impregnation step was omitted and the run temperature was 800°C. The solid was contacted with methane as described in Example 2. Results are reported below in Table VIII.

## TABLE VIII

| Run time (min) | % Conversion | % Selectivity | | | | |
|---|---|---|---|---|---|---|
| | | $CH_2CH_2$ | $CH_3CH_3$ | $C_3+$ | CO | $CO_2$ |
| Instantaneous Results* | | | | | | |
| 1 | 23.9 | 17.6 | 37.4 | 4.9 | 15.6 | 47.3 |
| 2 | 8.51 | 37.9 | 38.4 | 0 | 13.6 | 31.1 |
| 4 | 3.29 | 53.4 | 32.5 | 0 | 14.0 | 15.26 |
| 12 | 0.48 | 60.0 | 40.0 | 0 | 0 | – |
| 30 | 0.36 | 41.1 | 58.9 | 0 | – | – |
| Cumulative Results | | | | | | |
| 30 | 2.08 | 27.8 | 8.3 | – | 17.9 | 25.9 |

## EXAMPLE 3

A contact solid comprising a reducible oxide of manganese and an alkali metal component was prepared by impregnating Dart (Registered Trade Mark) magnesia with sodium permanganate to yield a solid containing the equivalent of 10 wt.% $NaMnO_2/Mg\ O$. The impregnated solids were dried at 100°C for 2 hours and then calcined in air at 1000°C for 16 hours. A quartz tube reactor (12 mm inside diameter) was charged with 7 ml. of solid. The solid was then subjected to 14 cycles comprising methane contact and air regeneration. The results obtained during the fourteenth are shown below in cable IX. These results were obtained at a reaction temperature of 825°C, at about atmospheric pressure and at a GHSV of 2400 hr.$^{-1}$. The results shown are based on analysis of a sample accumulated during a run time of about 2 minutes. The solid had not previously been contacted with a halogen source.

At the end of Run 14, the solid was contacted with methylene chloride by bubbling $N_2$ through $CH_2Cl_2$ and passing the resulting gas over the solid at temperatures up to 600°C. The solid was then regenerated in air for 30 minutes at temperatures up to 800°C. Methane conversion results obtained during subsequent cycles of methane conversion are shown in Table IX below. Following contact with methylene chloride, performance improved up to Run 23. The results shown are based on an analyses of samples, accumulated during a methane contact time of about 2 minutes.

TABLE IX

| Run# | T (°C) | GHSV (hr.$^{-1}$) | % Conver. | % Selectivity | | | | | |
|------|--------|-------------------|-----------|---------------|------|--------|-----|--------|------|
| | | | | $C_2H_4$ | $C_2H_6$ | $C_3^-$ | CO | $CO_2$ | Coke |
| 14 | 825 | 2400 | 9.04 | 31.7 | 34.0 | 6.2 | 0.5 | 21.2 | ? . |
| 15 | 825 | 1200 | 11.6 | 20.7 | 1.9 | 3.1 | 5.2 | 11.4 | 47.8 |
| 16 | 825 | 1200 | 17.0 | 33.8 | 4.8 | 8.5 | 2.6 | 37.2 | 13.1 |
| 19 | 825 | 1200 | 17.2 | 49.2 | 9.5 | 16.2 | 1.9 | 21.7 | 1.4 |
| 21 | 825 | 1200 | 16.7 | 49.6 | 10.7 | 14.5 | 2.1 | 22.8 | 0.4 |
| 22 | 825 | 1200 | 21.4 | 50.7 | 8.7 | 15.7 | 2.2 | 23.0 | 0.2 |
| 23 | 825 | 2400 | 8.2 | 44.4 | 28.4 | 8.6 | 1.4 | 17.1 | 0.2 |
| 24 | 825 | 600 | 25.1 | 35.0 | 6.2 | 10.7 | 1.9 | 46.2 | 0.1 |
| 26 | 825 | 1200 | 13.8 | 41.1 | 14.8 | 9.7 | 1.8 | 32.5 | 0.1 |

## EXAMPLE 4

A quartz tube reactor was charged wth 10 ml.(7.66gm) of 12-28 mesh (0.589 to 1.397mm) particles consisting of the equivalent of 15 wt. % Mn/5 wt. % $Na_4P_2O_7$/silica. The solid was prepared by impregnating the silica support with appropriate amounts of sodium pyrophosphate and manganese (as manganese acetate). The solid was then subjected to a number of cycles comprising methane contact and air regeneration. Results obtained are summarized below in Table X.

Several runs (Runs 3-10 in Table X) were made with a pure methane feed using the cyclic process wherein feed is passed over the solid for 2 minutes, followed by a 10 minute $N_2$ purge, a 20 minute air reoxidation, and a 15 minute $N_2$ purge. The effect of halogen on methane conversion was demonstrated by exposure to methyl chloride during the methane reaction portion of the cycle. A feed consisting of 5 vol. % $CH_3Cl$ and 95 vol. % $CH_4$ was used in Runs 14-17. The conversion at 750°C (Run #17) was substantially greater than the conversion at 800°C with pure methane feed (Run #7). The $C_2+$ selectivity of these two runs was similar. The effect of halogen treatment became more evident during runs which followed $CH_3Cl$ exposure. In Run 18, conversion remained the same while $C_2+$ selectivity increased. The initial halogen effect lasted for several runs (see Runs 18-37 in Table X). Although the activity of the solid decreased over the course of these runs, the $C_2+$ selectivity remained very high. Increasing the reaction temperature allowed some of the "lost" activity to be regained (see Runs 38-44). By again exposing the solid to a feed containing 5 vol. % $CH_3Cl$ in $CH_4$, activity was fully restored (see Runs 45 and 46) and pure methane runs following this exposure again showed high conversion and $C_2+$ selectivity (see Run 47-53).

## TABLE X

| Run# | Temp (°C) | Feed | Total GHSV | % Conv | % Selectivity To: $C_2+$ | CO | $CO_2$ | Coke |
|------|-----------|------|------------|--------|------|------|--------|------|
| 3 | 800 | $CH_4$ | $600hr^{-1}$ | 30.9 | 54.0 | 12.8 | 32.7 | 0.5 |
| 4 | 800 | $CH_4$ | $600hr^{-1}$ | 30.5 | 58.0 | 12.7 | 28.5 | 0.7 |
| 5 | 825 | $CH_4$ | $600hr^{-1}$ | 40.5 | 47.3 | 15.5 | 35.7 | 0.5 |
| 6 | 825 | $CH_4$ | $600hr^{-1}$ | 40.3 | 48.1 | 15.2 | 36.0 | 0.7 |
| 7 | 800 | $CH_4$ | $860hr^{-1}$ | 21.5 | 69.1 | 11.8 | 18.5 | 0.6 |
| 8 | 800 | $CH_4$ | $860hr^{-1}$ | 19.4 | 72.8 | 10.5 | 16.0 | 0.7 |
| 9 | 800 | $CH_4$ | $1200hr^{-1}$ | 13.2 | 77.4 | 10.3 | 11.0 | 1.3 |
| 10 | 800 | $CH_4$ | $1200hr^{-1}$ | 12.2 | 78.1 | 11.2 | 10.1 | 0.6 |
| 14 | 700 | 95%$CH_4$, 5%$CH_3Cl$ | $600hr^{-1}$ | 12.7 | 67.0 | 13.5 | 17.7 | 1.8 |
| 15 | 750 | 95%$CH_4$, 5%$CH_3Cl$ | $600hr^{-1}$ | 27.7 | 70.2 | 8.1 | 19.3 | 2.4 |

## TABLE X (Continued)

| Run# | Temp (°C) | Feed | Total GHSV | % Conv | % C₂+ | % CO | % CO₂ | % Coke |
|------|-----------|------|------------|--------|-------|------|-------|--------|
| 16 | 750 | 95%$CH_4$, 5%$CH_3Cl$ | $600hr^{-1}$ | 35.5 | 69.9 | 7.0 | 18.3 | 4.7 |
| 17 | 750 | 95%$CH_4$, 5%$CH_3Cl$ | $900hr^{-1}$ | 27.1 | 67.2 | 7.1 | 18.5 | 7.2 |
| 18 | 750 | $CH_4$ | $900hr^{-1}$ | 27.2 | 84.8 | 0.9 | 5.9 | 8.4 |
| 19 | 750 | $CH_4$ | $900hr^{-1}$ | 27.8 | 86.2 | 1.2 | 4.7 | 7.8 |
| 21 | 750 | $CH_4$ | $900hr^{-1}$ | 30.1 | 85.9 | 3.4 | 6.7 | 4.0 |
| 22 | 750 | $CH_4$ | $900hr^{-1}$ | 31.8 | 86.6 | 3.6 | 5.9 | 3.8 |
| 25 | 750 | $CH_4$ | $900hr^{-1}$ | 30.9 | 90.0 | 3.3 | 5.1 | 1.6 |
| 30 | 750 | $CH_4$ | $900hr^{-1}$ | 25.7 | 93.1 | 2.5 | 3.4 | 1.0 |
| 34 | 750 | $CH_4$ | $900hr^{-1}$ | 17.8 | 93.3 | 2.8 | 3.1 | 0.8 |
| 37 | 750 | $CH_4$ | $900hr^{-1}$ | 14.4 | 93.5 | 2.8 | 2.9 | 0.9 |
| 38 | 775 | $CH_4$ | $900hr^{-1}$ | 20.8 | 94.5 | 0.8 | 4.0 | 0.7 |
| 41 | 800 | $CH_4$ | $900hr^{-1}$ | 23.2 | 85.7 | 4.0 | 9.4 | 0.8 |
| 44 | 800 | $CH_4$ | $900hr^{-1}$ | 20.3 | 82.0 | 4.7 | 12.0 | 1.3 |
| 45 | 750 | 95%$CH_4$, 5%$CH_3Cl$ | $900hr^{-1}$ | not determined | | | | |
| 46 | 750 | 95%$CH_4$, 5%$CH_3Cl$ | $900hr^{-1}$ | 25.8 | 71.8 | 7.2 | 17.5 | 3.5 |
| 47 | 750 | $CH_4$ | $900hr^{-1}$ | 26.4 | 88.9 | 3.1 | 5.7 | 2.3 |
| 48 | 750 | $CH_4$ | $900hr^{-1}$ | 29.4 | 86.5 | 3.3 | 7.9 | 2.2 |
| 49 | 750 | $CH_4$ | $900hr^{-1}$ | 29.8 | 88.2 | 3.1 | 6.6 | 2.1 |
| 50 | 750 | $CH_4$ | $900hr^{-1}$ | 29.5 | 90.1 | 3.2 | 4.7 | 2.1 |
| 52 | 750 | $CH_4$ | $900hr^{-1}$ | 27.8 | 92.7 | 3.4 | 3.6 | 0.9 |
| 53 | 750 | $CH_4$ | $900hr^{-1}$ | 26.4 | 93.5 | 2.5 | 3.2 | 0.8 |

## Claims

1. A method for converting methane to higher hydrocarbon products by contacting a gas comprising, methane at a temperature in the range of 500° C to 1000° C with a contact solid comprising at lease one reducible oxide (as herein defined) of at least one metal which oxide when contacted with methane at a temperature in said range is reduced and produces higher hydrocarbon products and water, characterised in that said contacting is effected in the presence of at least one promotor selected from halogens, chalcogens and compounds thereof, said chalcogens being selected from sulfur, selenium and tellurium.

2. A method as claimed in Claim 1 characterised in that said promoter is included in said contact solid.

3. A method as claimed in Claim 1 characterised in that said promoter is provided by at least periodically contacting said contact solid with at least one source selected from halogen and chalcogen sources.

4. A method as claimed in Claim 1 characterised in that it comprises the steps of:
   (a) contacting a gas comprising methane with a contact solid comprising at least one said reducible oxide of at least one metal under conditions at which said at least one reducible oxide is reduced to form solids comprising reduced metal oxide and produces higher hydrocarbon products and water;
   (b) recovering higher hydrocarbons;
   (c) at least periodically contacting solids comprising reduced metal oxide with an oxygen containing gas to regenerate solids comprising reducible metal oxide;
   (d) contacting a gas comprising methane with regenerated solids produced in step (c) in the manner specified in step (a); and
   (e) at least periodically contacting said solids comprising reduced metal oxide and/or said regenerated solids comprising reducible metal oxide with the promoter source.

5. A method as claimed in claim 1 characterised in that it includes the further steps of:
   (a) recovering higher hydrocarbons produced by said contacting;
   (b) at least periodically contacting solids comprising reduced metal oxide, and obtained by said reduction of said at least one reducible oxide, with an oxygen-containing gas to regenerate solids comprising reducible metal oxide;
   (c) contacting a gas comprising methane with regenerated solids produced in step (b) in the manner specified in claim 1; and
   (d) at least periodically contacting said solids comprising reduced metal oxide and/or said regenerated solids comprising reducible metal oxide with at least one promoter source selected from halogen sources and chalcogen sources to incorporate additional promoter into said solids.

6. A method as claimed in claim 4 or claim 5 characterised in that said oxygen-containing gas and said promoter source are simultaneously contacted with solids comprising reduced metal oxides and/or said gas comprising methane and said promoter source are simultaneously contacted with solids comprising reducible metal oxides.

7. A method as claimed in any one of claims 1 to 6 characterised in that said contact solid further comprises at least one phosphorus component.

8. A method as claimed in any one of claims 1 to 7 characterised in that said at least one reducible oxide is selected from reducible oxides of Mn, Sn, In, Ge, Pb, Sb and Bi, and preferably from reducible oxides of Mn.

9. A method as claimed in any one of claims 1 to 8 characterised in that said contact solid satisfies the emperical formula

   $$A_a \, X_b \, P_c \, O_d$$

   where A is selected from Mn, Sn, In, Ge, Pb, Sb, Bi and mixtures thereof; X is selected from F, Cl, Br, I, S, Se, Te and mixtures thereof; a to d indicate the atomic ratio of each component and when a is 10, b is in the range 0.01 to 30, c- is in the range 0 to 20 and d has a value which is determined by the valence and proportions of the other elements present.

10. A method as claimed in any one of claims 1 to 7 characterised in that said at least one reducible oxide is selected from oxides of cerium, praseodymium, terbium, iron and ruthenium.

11. A method as claimed in, any one of claims 1 to 10 characterised in that said contact solid further comprises at least one member selected from alkali metals, alkaline earth metals and compounds thereof.

12. A method as claimed in any of Claims 1 to 11 characterised in that the contact solid is associated with a support material.

15

13. A method as claimed in Claim 12 characterised in that the contact solid is selected from silica- and magnesia-supported halogen-promoted solids containing oxides of manganese and sodium.

**Revendications**

1. Procédé pour convertir du méthane en produits hydrocarbonés supérieurs par mise en contact d'un gaz comprenant du méthane á une température de l'ordre de 500° à 1000°C avec un solide de contact comprenant au moins un oxyde réductible [tel que défini ici] d'au moins un métal, lequel oxyde lorsqu'il est mis en contact avec du méthane à une température de cet ordre est réduit et fournit des produits hydrocarbonés supérieurs et de l'eau caractérisé en ce que ce contact est effectué en présence d'au moins un activateur choisi parmi des halogènes, des chalcogènes et leurs composés, ces chalcogènes étant choisis parmi le soufre, le sélénium et le tellure.

2. Procédé suivant la revendication 1, caractérisé en ce que l'activateur est inclus dans ce solide de contact.

3. Procédé suivant la revendication 1, caractérisé en ce que cet activateur est fourni par mise en contact de façon au moins périodique de ce solide de contact avec au moins une source choisie parmi des sources d'halogène et de chalcogène.

4. Procédé suivant la revendication 1, caractérisé en ce qu'il comprend les étapes suivantes:
   [a] mise en contact d'un gaz comprenant du méthane avec un solide de contact comprenant au moins un tel oxyde réductible d'au moins un métal dans des conditions dans lesquelles cet oxyde réductible au moins présent est réduit pour former des solides comprenant de l'oxyde métallique réduit et fournit des produits hydrocarbonés supérieurs et de l'eau;
   [b] récupération des hydrocarbures supérieurs;
   [c] mise en contact de façon au moins périodique des solides comprenant de l'oxyde métallique réduit avec un gaz contenant de l'oxygène pour régénérer les solides comprenant l'oxyde métallique réductible;
   [d] mise en contact d'un gaz comprenant du méthane avec les solides régénérés produits dans l'étape [c] selon la procédure de l'étape [a]; et
   [e] mise en contact de façon au moins périodique de ces solides comprenant de l'oxyde métallique réduit et/ou de ces solides régénérés comprenant de l'oxyde métallique réductible avec la source d'activateur.

5. Procédé suivant la revendication 1, caractérisé en ce qu'il comprend de plus les étapes suivantes;
   [a] récupération des hydrocarboure supérieurs produits par ce contact;
   [b] mise en contact de façon au moins périodique des solides comprenant de l'oxyde métallique réduit et obtenu par cette réduction de cet oxyde réductible au moins présent, avec un gaz contenant de l'oxygène pour régénérer des solides comprenant de l'oxyde métallique réductible;
   [c] mise en contact d'un gaz comprenant du méthane avec les solides régénérés produits dans l'étape [b] selon la procédure décrite dans la revendication 1; et
   [d] mise en contact de façon au moins périodique de ces solides comprenant de l'oxyde métallique réduit et/ou de ces solides régénérés comprenant de l'oxyde métallique réductible avec au moins une source d'activateur choisie parmi des sources d'halogène et de chalcogène pour incorporer de l'activateur supplémentaire dans ces solides.

6. Procédé suivant la revendication 4 ou la revendication 5, caractérisé en ce que ce gaz contenant de l'oxygène et cette source d'activateur sont simultanément mis en contact avec des solides comprenant des oxydes métalliques réduits et/ou que ce gaz contenant du méthane et cette source d'activateur sont simultanément mis en contact avec des solides comprenant des oxydes métalliques réductibles.

7. Procédé suivant l'une quelconque des revendications 1 à 6. caractérisé en ce que ce solide de contact comprend de plus au moins un composé du phosphore.

8. Procédé suivant l'une quelconque des revendications 1 à 7, caractérisé en ce que cet oxyde rèductible au moins présent est choisi parmi des oxydes réductibles de Mn, Sn, In, Ge, Pb, Sb et Bi et de

EP 0 179 857 B1

préférence parmi des oxydes réductibles de Mn.

9. Procédé suivant l'une quelconque des revendications 1 à 8, caractérisé en ce que ce solide de contact satisfait à la formule empirique;

$$A_aX_bP_cO_d$$

dans laquelle A est choisi parmi Mn, Sn, In, Ge, Pb, Sb, Bi et leurs mélanges;X est choisi parmi F, Cl, Br, I, S, Se, Te et leurs mélanges; a à d indiquent le rapport atomique de chaque composant et lorsque a est égal à 10, b est dans la gamme de 0,01 à 30, c est dans la gamme de 0 à 20 et d présente une valeur qui est déterminée par la valence et les proportions des autres éléments présents.

10. Procédé suivant l'une quelconque des revendications 1à 7. caractérisé en ce que cet oxyde réductible au moins présent est choisi parmi des oxydes de cérium, de praséodyme de terbium, de fer et de ruthénium.

11. Procédé suivant l'une quelconque des revendications 1 à 10. caractérisé en ce que ce solide de contact comprend de plus au moins un membre choisi parmi des métaux alcalins, des métaux alcalino-terreux et leurs composés.

12. Procédé suivant l'une quelconque des revendications 1 à 11. caractérisé en ce que ce solide de contact est associé à un matériau de support.

13. Procédé suivant la revendication 12, caractérisé en ce que ce solide de contact est choisi parmi des solides contenant des oxydes de manganèse et de sodium sur support de silice et de magnésie, activés par un halogène.


## Ansprüche

1. Verfahren zur Umwandlung von Methan in höhere Kohlenwasserstoff-Produkte durch In-Kontakt-Bringen eines Methan umfassenden Gases bei einer Temperatur im Bereich von 500 °C bis 1000 °C mit einem Kontakt-Feststoff, der wenigstens ein reduzierbares Oxid (gemäß nachstehender Definition) von wenigstens einem Metall umfaßt, wobei das Oxid bei Kontakt mit Methan bei einer Temperatur in dem genannten Bereich reduziert wird und höhere Kohlenwasserstoff-Produkte und Wasser erzeugt, dadurch gekennzeichnet, daß das In-Kontakt-Bringen In Gegenwart wenigstens eines Promoters, ausgewählt aus Halogenen, Chalcogenen und deren Verbindungen bewirkt wird, wobei die Chalcogene gewählt sind unter Schwefel, Selen und Tellur.

2. Verfahren nach Anspruch 1, dadurch gekennzeichnet, daß der Promoter in dem Kontakt-Feststoff eingeschlossen ist.

3. Verfahren nach Anspruch 1, dadurch gekennzeichnet, daß der Promoter dadurch bereitgestellt wird, daß man den Kontakt-Feststoff wenigstens periodisch mit wenigstens einer Ausgangssubstanz in Kontakt bringt, die gewählt ist aus Halogen-und Chalcogen-Ausgangssubstanzen.

4. Verfahren nach Anspruch 1, dadurch gekennzeichnet, daß es folgende Schritte umfaßt:
(a) In-Kontakt-Bringen eines Methan umfassenden Gases mit einem Kontakt-Feststoff, der wenigstens ein reduzierbares Oxid wenigstens eines Metalls umfaßt, unter Bedingungen, bei denen wenigstens ein reduzierbares Oxid unter Bildung von reduziertes Metalloxid umfassenden Feststoffen reduziert wird und höhere Kohlenwasserstoff-Produkte und Wasser erzeugt;
(b) Gewinnen höherer Kohlenwasserstoffe;
(c) wenigstens periodisches In-Kontakt-Bringen von reduziertes Metalloxid umfassenden Feststoffen mit einem Sauerstoff enthaltenden Gas, um die reduzierbares Metalloxid umfassenden Feststoffe, zu regenerieren;
(d) In-Kontakt-Bringen eines Methan umfassenden Gases mit regenerierten, in Schritt (c) in der in Schritt (a) angegebenen Weise hergestellten Feststoffen; und
(e) wenigstens periodisches In-Kontakt-Bringen der reduziertes Metalloxid umfassenden Feststoffe und/oder der reduzierbares Metalloxid umfassenden regenerierten Feststoffe mit der Promoter-

17

Ausgangssubstanz.

5. Verfahren nach Anspruch 1, dadurch gekennzeichnet, daß es die folgenden weiteren Schritte umfaßt:
   (a) Gewinnen der durch das In-Kontakt-Bringen erzeugten höheren Kohlenwasserstoffe;
   (b) wenigstens periodisches In-Kontakt-Bringen der reduziertes Metalloxid umfassenden und durch die Reduktion des wenigstens einen reduzierbaren Oxids erhaltenen Feststoffe mit einem Sauerstoff enthaltenden Gas unter Regenerierung von reduzierbares Metalloxid umfassenden Feststoffen;
   (c) In-Kontakt-Bringen eines Methan umfassenden Gases mit in Schritt (b) in der in Anspruch 1 angegebenen Weise hergestellten regenerierten Feststoffen; und
   (d) wenigstens periodisches In-Kontakt-Bringen der reduziertes Metalloxid umfassenden Feststoffe und/oder der reduzierbares Metalloxid umfassenden regenerierten Feststoffe mit wenigstens einer aus Halogen-Ausgangssubstanzen oder Chalcogen-Ausgangssubstanzen gewählten Promoter-Ausgangssubstanz, um zusätzlichen Promoter in die Feststoffe einzubringen.

6. Verfahren nach Anspruch 4 oder Anspruch 5, dadurch gekennzeichnet, daß das Sauerstoff enthaltende Gas und die Promoter-Ausgangssubstanz gleichzeitig mit reduzierte Metalloxide umfassenden Feststoffen in Kontakt gebracht werden und/oder das Methan umfassende Gas und die Promoter-Ausgangssubstanz gleichzeitig mit den reduzierbare Metalloxide umfassenden Feststoffen in Kontakt gebracht werden.

7. Verfahren nach einem der Ansprüche 1 bis 6, dadurch gekennzeichnet, daß der Kontakt-Feststoff außerdem wenigstens eine Phosphorkomponente enthält.

8. Verfahren nach einem der Ansprüche 1 bis 7, dadurch gekennzeichnet, daß das wenigstens eine reduzierbare Oxid gewählt ist aus reduzierbaren Oxiden von Mn, Sn, In, Ge, Pb, Sb und Bi und vorzugsweise aus reduzierbaren Oxiden von Mn.

9. Verfahren nach einem der Ansprüche 1 bis 8, dadurch gekennzeichnet, daß der Kontakt-Feststoff der empirischen Formel

   $A_a X_b P_c O_d$

   genügt, worin A gewählt ist aus Mn, Sn, In, Ge, Pb, Sb, Bi und Mischungen davon; X gewählt ist aus F, Cl, Br, I, S, Se, Te und Mischungen davon; a bis d das Atomverhältnis jeder Komponente angeben, und wenn a gleich 10 ist, b im Bereich von 0,01 bis 30 liegt, c im Bereich von 0 bis 20 liegt und d den Wert hat, der durch die Valenzen und Verhältnisse der anderen anwesenden Elemente bestimmt wird.

10. Verfahren nach einem der Ansprüche 1 bis 7, dadurch gekennzeichnet, daß das wenigstens eine reduzierbare Oxid gewählt ist aus Oxiden von Cer, Praseodym. Terbium, Eisen und Ruthenium.

11. Verfahren nach einem der Ansprüche 1 bis 10, dadurch gekennzeichnet, daß der Kontakt-Feststoff außerdem wenigstens ein Element gewählt aus der Gruppe Alkalimetalle, Erdalkalimetalle und deren Verbindungen umfaßt.

12. Verfahren nach einem der Ansprüche 1 bis 11, dadurch gekennzeichnet, daß der Kontakt-Feststoff mit einem Trägermaterial verbunden ist.

13. Verfahren nach Anspruch 12, dadurch gekennzeichnet, daß der Kontakt-Feststoff gewählt ist aus auf Siliciumoxid- und Magnesiumoxid-Trägern aufgezogenen, Halogen-unterstützten Feststoffen, welche Oxide von Mangan und Natrium enthalten.